# EUROPEAN PATENT APPLICATION

(11) **EP 1 495 765 A1**
(43) Date of publication of application: **12.01.2005**
(21) Application number: 03719100.4
(22) Date of filing: 11.04.2003
(51) Int. Cl.: A61K 35/78, A61P 9/10, A23L 1/30, A23L 1/36

(54) **COMPOSITION FOR PREVENTING ARTERIOSCLEROSIS**

(30) Priority: 12.04.2002 JP 2002110932
(71) Applicant: Ajinomoto Co., Inc., Tokyo 104-8315 (JP)
(72) Inventor: KOYAMA, Naoto, c/o Ajinomoto Co., Inc., Kawasaki-shi, Kanagawa 210-8681 (JP); KURIBAYASHI, Kanna, c/o Ajinomoto Co,, Inc., Kawasaki-shi, Kanagawa 210-681 (JP); ISHII, Koichi, c/o Ajinomoto Co., Inc., Kawasaki-shi, Kanagawa 210-8681 (JP); KOBAYASHI, Katsunori, c/o Ajinomoto Co., Inc., Kawasaki-shi, Kanagawa 210-8681 (JP)
(74) Representative: Nicholls, Kathryn Margaret
(86) International application number: PCT/JP2003/004607
(87) International publication number: WO 2003/086437

(57) **Abstract**

A superior composition for preventing atherosclerosis, and a food and a pharmaceutical composition containing the composition are provided.

A composition obtained by organic solvent extraction of defatted plant seed, and a food and a pharmaceutical composition containing the composition are provided.

## Description

### Technical Field

The present invention relates to a composition for preventing atherosclerosis, which can be obtained by organic solvent extraction of plant seeds after defatting, and a food and a pharmaceutical composition containing the composition for preventing atherosclerosis.

### Background Art

Along with the westernization of life style in recent years, in addition to cancer, atherosclerotic diseases such as angina pectoris, intermittent claudication, cardiac infarction, cerebral infarction and the like have become the main causes of death of Japanese people. Once developed, these diseases are very difficult to cure, and dramatically degrade QOL (quality of life). It is undisputable that a countermeasure to prevent or control the progression of the disease state thereof is highly extremely important from a social standpoint as well. A consensus has been generally reached that oxidation of LDL (low density lipoprotein) plays a key role in the early stages of lesion formation, the importance of not only controlling the blood cholesterol level to a suitable range but also suppressing the production of oxidized LDL has been pointed out recently. It has been clarified that foods, particularly a food derived from plants, contain an abundance of anti-oxidative substances, and the anti-oxidative substances contained in green tea and red wine are considered to be taken into LDL (or in the vicinity thereof) and eliminate radical to prevent production of oxidized LDL (Fuhrman et al, Am. J. Clin. Nutr., 61: pp 549-54, 1995). There is also an epidemiological study that concludes that positive intake of these foods suppress cancer and heart diseases (Renaud et al, Lancet, 339: pp 1523-26, 1992).

In the meantime, there is a report that particular components derived from particular seeds such as sesame seed lignan, grapeseed polyphenol and the like show an anti-atherosclerotic activity with experimental animals (Kang et al, J. Nutr., 129: pp 1885-90, 1999) (Yamakoshi et al, Atherosclerosis 142: pp 139-49, 1999). However, the anti-atherosclerotic property of a plant seed component has been clarified at an animal test level only in a few cases, and many researches remain at a test tube level. For example, JP-A-8-337536 discloses an anti-active oxygen agent extracted from a roasted and then fermented plant seed. The technique uses a plant seed as a starting material, but has low versatility because it requires operations such as roasting, fermentation treatment and the like, and, thus, this technique is not practical. In addition, the effect of suppressing atherosclerosis is not clearly shown. Furthermore, Zhang et al (Chem. Pharm. Bull., 45: pp 1910-14, 1997) report structures of a group of compounds extracted from a safflower oil cake by distribution of various solvents, and that some of these structures have an antioxidant activity in vitro. However, it is not clear at present if such compounds having an antioxidant activity are effective for preventing atherosclerosis. In consideration of the fact that antioxidant activity in vitro is known not to be necessarily correlated to the anti-atherosclerotic activity in living organisms (Fruebis et al, J. Lipid Res., 38: pp 2455-64, 1997, Fruebis et al, Atherosclerosis 117: pp 217-24, 1995, Munday et al, Arterioscler. Thromb. Vasc. Biol., 18: pp 114-19, 1998, Wagberg et al, J. Pharmacol. Exp. Ther., 299: pp 76-82, 2001), confirmation of whether or not an anti-oxidative substance in a plant seed has anti-atherosclerotic property is required at least at an experimental animal level.

### Disclosure of the Invention

An object of the present invention is to provide a composition effective for preventing atherosclerosis, which is one of the lifestyle-related diseases, and a food and a pharmaceutical composition containing this composition.

The present inventors have conducted intensive studies to achieve the above-mentioned object and found that a composition extracted with organic solvent from defatted plant seeds has not only an antioxidant activity in vitro but also an atherosclerosis preventive activity in experimental animals. On the basis of these findings, the present invention has been completed.

Thus, the present invention provides the following. That is:
(1) A composition for preventing atherosclerosis, which can be obtained by organic solvent extraction of defatted plant seeds.
(2) The composition of the above-mentioned (1), wherein the plant seed is a seed of safflower or rapeseed.
(3) The composition of the above-mentioned (1) or (2), wherein the organic solvent extraction comprises extracting defatted plant seeds with lower alcohol.
(4) The composition of the above-mentioned (3), wherein the organic solvent extraction comprises extraction with acetate ester after the extraction with lower alcohol.
(5) The composition of the above-mentioned (3) or (4), wherein the organic solvent extraction comprises, after the extraction with lower alcohol, evaporating lower alcohol, adding water and washing an aqueous phase with a nonpolar solvent.
(6) The composition of any of the above-mentioned (3) to (5), wherein the lower alcohol is ethanol or methanol.
(7) The composition of any of the above-mentioned (4) to (6), wherein the acetate ester is ethyl acetate, methyl acetate or propyl acetate.
(8) The composition of any of the above-mentioned (5) to (7), wherein the nonpolar solvent is n-hexane.
(9) A food comprising the composition of any of the above-mentioned (1) to (8).
(10) A pharmaceutical composition comprising the composition of any of the above-mentioned (1) to (8).

### Brief Description of Figures

Fig. 1 is a graph showing an effect on the oxidizability of LDL in each sample of Example 1.
Fig. 2 is a photograph showing that a safflower meal extract composition and a rapeseed meal extract composition have a suppressive effect on atherosclerosis in the aorta of apoE (-/-) mice [atherosclerosis model mice](week 5 of administration, 14-week-old) in Example 2, wherein (a) is a control group, (b) is a safflower group and (c) is a rapeseed group.
Fig. 3 is a sketch of Fig. 2, wherein (a-1) is a sketch of Fig. 2(a), (b-1) is a sketch of Fig. 2(b), (c-1) is a sketch of Fig. 2(c). The photograph of Fig. 2 is originally a color photograph and the part stained in red shows an atheromatous plaque. Fig. 3 is a sketch for the purpose of clarifying the red stained part in Fig. 2 by drawing a figure of Fig. 2 and blacked out the red stained part.
Fig. 4 is a graph showing an oxidation curve of LDL induced by the addition of V70 (lipid peroxide producing curve).
Fig. 5 is a graph showing an aortic root lesion area in apoE knockout mice (21-week-old, male, week 15 of administration of each sample) in Example 3, wherein SFM is a safflower meal extract composition, CS is p-coumaroylserotonin and FS is feruloylserotonin.

### Detailed Description of the Invention

The plant seed to be used in the present invention may be a seed of any plant, and, for example, seeds of, for example, safflower, rapeseed, soybean and the like can be mentioned, with preference given to the seeds of safflower and rapeseed. In the present invention, a plant seed means the whole constituting a plant seed, or a part thereof, such as seed coat, albumen, germ and the like, or a mixture thereof.

In the present invention, plant seed after defatting, or a defatted material (meal), is used as a starting material. A defatted material of plant seed can be obtained by delipidating the plant seed by a method known per se. For example, the material can be obtained by press-extracting seeds or adding n-hexane and the like to a crushed seed, extracting the mixture, taking out a solid content from the extraction system and drying the solid content. The degree of defatting is generally not less than 60%, preferably not less than 80%.

The "organic solvent extraction" used in the present specification is explained below. Examples of the solvent to be used in the first step for extracting, by organic solvent extraction, from defatted seeds in the present invention include, but are not limited to, a lower alcohol (including water-containing lower alcohol), acetone (including water-containing acetone), acetonitrile (including water-containing acetonitrile), a mixed solvent thereof and the like. Preferred is a lower alcohol. As the lower alcohol, for example, alcohol having 1 to 4 carbon atoms can be mentioned. Specific examples thereof include, but are not limited to, methanol, ethanol, n-propanol, isopropanol, n-butanol and the like. The lower alcohol is preferably ethanol or methanol (including water-containing ethanol and water-containing methanol). A composition extracted with such solvent is useful as a composition of the present invention at a purity thereof, but may be processed to have a higher purity (may be purified).

One example for increasing the purity (purifying) is described in the following, but the method is not limited thereto. An organic solvent of the above-mentioned solvent extract in the first step is evaporated (particularly evaporated under reduced pressure), water is added to the obtained extract to suspend the same, the suspension (aqueous phase) is washed with a nonpolar solvent, such as n-hexane, n-heptane, n-octane and the like, preferably n-hexane, and the aqueous layer after washing is extracted with a solvent that can extract an object composition in two separate layers, such as acetate ester, n-butanol and the like, preferably acetate ester, particularly preferably ethyl acetate, methyl acetate or propyl acetate. Then, the extract is washed with saturated brine and the like to produce an organic layer. When it is extracted with acetate ester, the organic layer is dehydrated over, for example, anhydrous magnesium sulfate and the like, and then concentrated under reduced pressure to give a solid (composition). Purification may be stopped in any step during the above, or any step may be omitted, or modified and the purification may be further continued. A multi-step extraction method, a counter current distribution method and the like can be also used, including changing the kind of the above-mentioned solvent.

When the composition of the present invention obtained by the above-mentioned method is used as a food or a pharmaceutical agent (atherosclerosis preventive agent etc.) and when the composition is present in a physiologically harmful solvent, the composition may be dried, or the dry product is dissolved, suspended or emulsified in a physiologically acceptable solvent before use. The form of a composition includes a liquid such as an aqueous solution and the like, a solid obtained by concentration under reduced pressure and drying, a solidified product such as a lyophilized product and the like.

The composition for preventing atherosclerosis of the present invention suppresses oxidation of LDL (low density lipoprotein) in human plasma in vitro. Particularly, a composition obtained from the seeds of safflower and rapeseed suppresses oxidation of LDL particularly strongly. What is to be particularly noted is that the composition of the present invention suppresses formation of atheromatous plaque on an inner wall of the blood vessels in mouse in vivo and showed an anti-atherosclerotic activity in experimental animal. From the above, the composition of the present invention is useful as a pharmaceutical agent for prevention of atherosclerosis and the like, as well as a food for preventing atherosclerosis.

The composition of the present invention prevents atherosclerosis and is useful for preventing diseases caused by atherosclerosis, such as angina pectoris, cardiac infarction, intermittent claudication, cerebral infarction and the like.

The "food" of the present invention means food in general, and includes a general food including health food, Food for Specified Health Use and Food with Nutrient Function Claims as defined in the Food with Health Claims System of the Health, Labor and Welfare Ministry, and encompasses supplements.

As the food or pharmaceutical composition, the above-mentioned composition itself of the present invention can be used. In addition, it is possible to use the above-mentioned composition of the present invention contained in various foods, for example, general food (including what is called health food) such as dressing, mayonnaise and the like. Moreover, the composition of the present invention can be prepared into tablet, pill, granule, fine granules, powder, pellet, capsule, solution, emulsion, suspension, syrup, troche and the like together with excipients (e.g., lactose, sucrose, starch etc.), and with flavoring, dye and the like, and used as Food with Health Claims such as Food for Specified Health Use, Food with Nutrient Function Claims and the like, supplement, pharmaceutical preparation (pharmaceutical composition) (mainly for oral use).

Particularly, in the case of a pharmaceutical composition, the composition can be prepared along with a pharmaceutically acceptable carrier (including additive). Examples of the pharmaceutically acceptable carrier include, but not limited to, excipients (e.g., lactose, sucrose, starch, D-mannitol etc.), binders (e.g., cellulose, sucrose, dextrin, hydroxypropyl cellulose, polyvinylpyrrolidone etc.), disintegrants (e.g., starch, carboxymethyl cellulose etc.), lubricants (e.g., magnesium stearate etc.), surfactants (e.g., sodium lauryl sulfate etc.), solvents (e.g., water, brine, soybean oil etc.), preservatives (e.g., p-hydroxybenzoic acid ester etc.) and the like, which are known to those of ordinary skill in the art.

While the amount of intake or dose of the composition for preventing atherosclerosis of the present invention varies depending on the purity of the composition, age, body weight and health condition of the subject and the like, generally 10 mg - 10 g, preferably 100 mg - 10 g, is preferably given or administered to an adult per day for the prevention of atherosclerosis, which is given once a day or in several portions a day.

Since the composition of the present invention uses plant seeds which are conventionally used for food and the like (particularly, seeds of safflower and rapeseed), which are used as a starting material of cooking oil, the toxicity is extremely low and side effects are scarcely observed.

### Examples

The present invention is explained in detail by referring to Examples, which are not to be construed as limitative.

### Example 1 (in vitro anti-oxidation data)

To defatted safflower meal (100 g) was added 500 ml of aqueous ethanol containing 90 vol% of ethanol, and the mixture was warmed and stirred in hot water bath at 60°C for 3 hr, and filtered. The solid content after filtration was subjected to a similar step once, and the obtained filtrates were combined and concentrated under reduced pressure to give 60 ml of concentrated solution. Water was added to the concentrated solution to make 200 ml and the contents were suspended, which was washed twice with 120 ml of n-hexane. The aqueous layer after washing was extracted twice with ethyl acetate (100 ml). The ethyl acetate extract solution was washed with saturated brine, the ethyl acetate layer was dried over anhydrous magnesium sulfate, filtered and concentrated under reduced pressure to give a solid (1.16 g). Simultaneously, defatted rapeseed meal, soybean meal, soybean germ meal and soybean seed coat were respectively subjected to a similar treatment, 1 ml of DMSO was added to 1/10 amount of an extract for dissolution and used as samples.

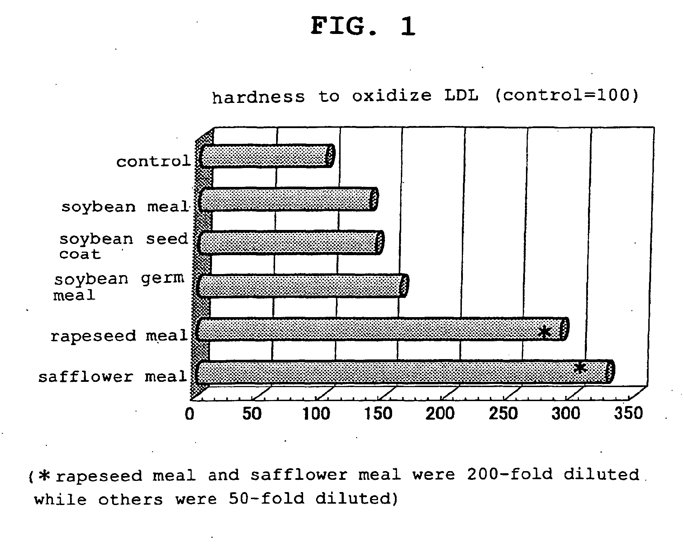

Plasma obtained from human volunteer (adjusted to density =1.21 (g/ml) with KBr) was applied to discontinuous density gradient centrifugation (417,000 x g, 40 min, 4°C) (OptimaTLX; Beckman Coulter) and an LDL band was withdrawn with a syringe. The protein content of the LDL fraction was measured (BCA protein assay kit; Pierce biotechnology, Inc.), and diluted with phosphate buffer (PBS) to a final concentration of 100 µg protein/ml. Thereto was added a 1/100 amount of the above-mentioned sample, and a radical initiator (V70; 2,2'-azobis(4-methoxy-2,4-dimethylvaleronitrile)) was added to a final concentration of 1 mM. The absorption at 234 nm (DU640; Beckman Coulter) based on the conjugated diene structure in lipid peroxide was immediately monitored for 5 hr. The lag time was calculated according to the method of Kondo et al. (J. Nutr. Sci. Vitaminol., 43: pp 435-44, 1997) based on the obtained lipid peroxide production curve. The effect of each sample on the oxidizability of LDL was evaluated by a relative value of the lag time with that of control (solvent alone was added) as 100 (Fig. 1). Every sample tended to more or less suppress oxidizability of LDL (i.e., extension of ragtime), but rapeseed meal and safflower meal particularly strongly suppressed the oxidization of LDL.

The dilution fold of each sample before mixing with diluted human LDL in the above-mentioned test was 200-fold for rapeseed meal and safflower meal, and 50-fold for others. Since these meal extracts are liquid, when converted to meal before extraction, rapeseed, and safflower meal extracts (200-fold diluted) were at 0.05 g/ml based on rapeseed meal and safflower meal, and soybean meal, soybean seed coat and soybean germ meal (50-fold diluted) were at 0.2 g/ml based on soybean meal, soybean seed coat and soybean germ meal. In other words, the rapeseed meal and safflower meal extract showed stronger antioxidant activity at a lower concentration.

### Example 2 (in vivo atherosclerosis preventive effect)

Extracts of rapeseed meal and safflower meal were prepared as follows.

To defatted rapeseed meal (600 g) was added 3000 ml of aqueous ethanol containing 90 vol% of ethanol, and the mixture was warmed and stirred in hot water bath at 60°C for 3 hr, and filtered. The solid content after filtration was subjected to a similar step once, and the obtained filtrates were combined and concentrated under reduced pressure to give 500 ml of concentrated solution. Water was added to the concentrated solution to make the amount 1000 ml and the contents were suspended, which was followed by washing twice with 500 ml of n-hexane. The aqueous layer after washing was extracted twice with ethyl acetate (500 ml). The ethyl acetate extract solution was dried over anhydrous magnesium sulfate, filtered and concentrated under reduced pressure to give an extract (12.5 g).

The defatted safflower meal (600 g) was treated in the same manner as above to give 10.1 g of an extract.

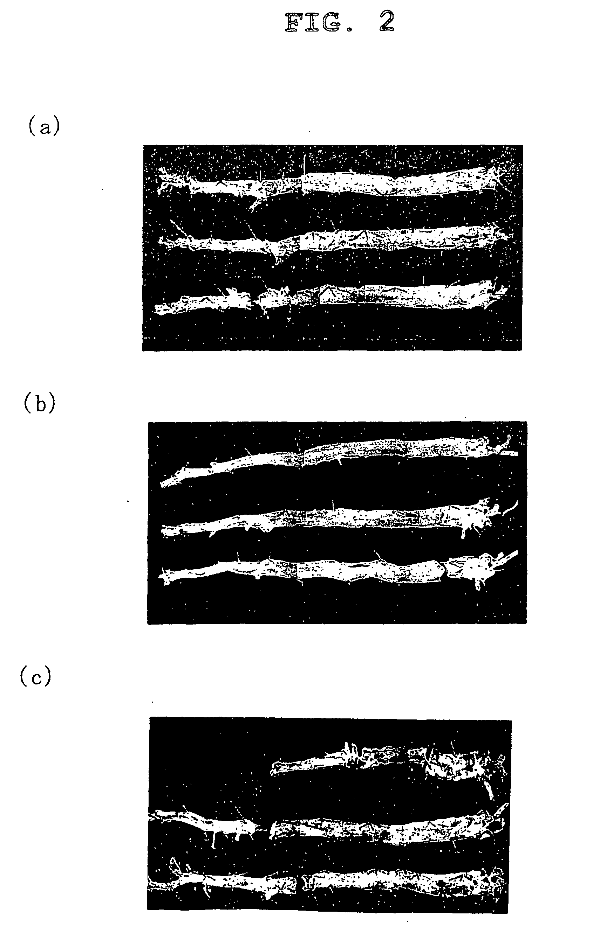

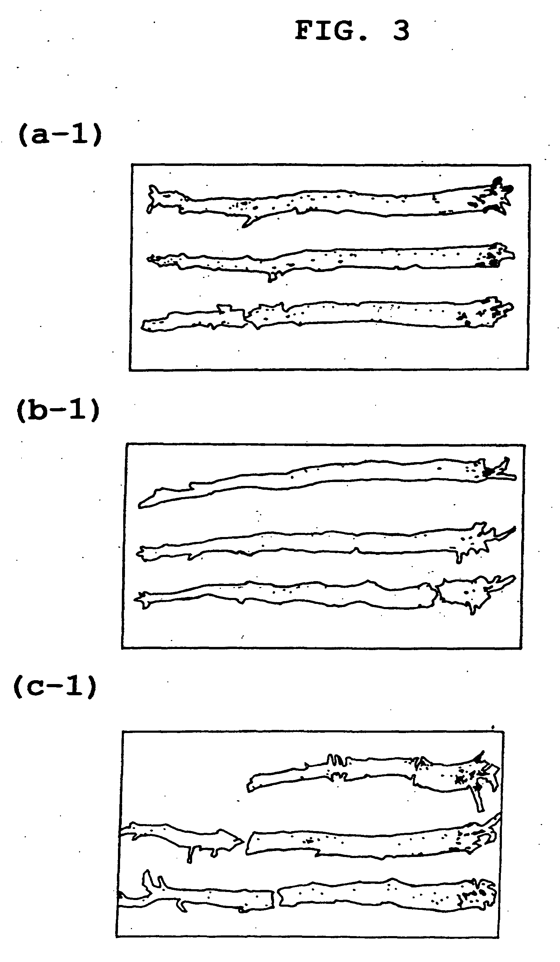

The 9-week-old male apoE knockout mice (apoE(-/-); purchased from The Jackson Laboratory) were divided into three groups of control/rapeseed (rapeseed meal extract administration group)/safflower (safflower meal extract administration group) with 9 mice per group, and each group was allowed free intake of a feed having ingredients shown in Table 1 for 5 weeks. The mice were killed at week 2 (n=6) and week 5 (n=3), a part of the aorta from the aortic root to the femoral artery bifurcation was removed, and the area of atheromatous plaque (plaque) formed on the vascular inner wall stained with Sudan IV was compared with that of the control group. By 2 weeks' administration, plaque formation was tended to be suppressed in the rapeseed group and safflower group as compared to the control group. By comparison of groups after extended administration for 3 weeks thereafter, the above-mentioned tendency became stronger (plaque area: safflower < rapeseed < control), and an effect of suppressing formation of initial lesion of atherosclerosis was exhibited by these oil plant meal extracts (Fig. 2 and Fig. 3).

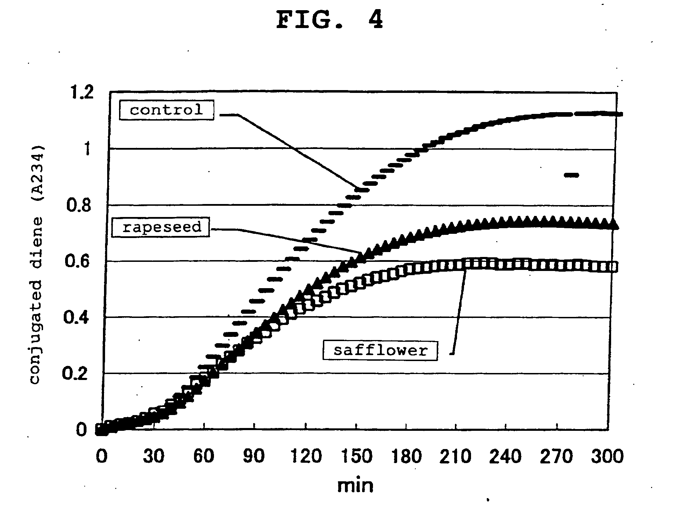

Blood was taken from the abdominal vena cava of the mouse killed in the above-mentioned test. Plasma (adjusted to density of 1.30 (g/ml) with KBr) separated according to conventional methods was subjected to discontinuous density gradient centrifugation (417,000 x g, 1 hr, 4°C)(Optima TLX; Beckman Coulter) and a lipoprotein fraction was fractionated using a fractionator (DGF-U; Hitachi Koki Co., Ltd.). The protein content of the VLDL (very low density lipoprotein) - LDL (low density lipoprotein) fraction of each group, confirmed by electrophoresis (Multigel-lipo; Daiichi Pure Chemicals Co., Ltd), was measured (BCA protein assay kit; Pierce Biotechnology, Inc.), the fraction was diluted with phosphate buffer (PBS) to a final concentration of 50 µg protein/ml, a radical initiator (V70; 2,2'-azobis(4-methoxy-2,4-dimethyl valeronitrile)) was added to a final concentration of 250 µM, and the measurement of absorption at 234 nm (DU-640; Beckman Coulter) based on the conjugated diene structure in lipid peroxide at 37°C was immediately initiated. A lipid peroxide production curve was plotted for each of the administration group and the control group and production rate and the amount of product were compared (Fig. 4). As a result, it was clarified that the lipoprotein fractions of the rapeseed and safflower administration groups showed lesser accumulation of lipid peroxide as compared to the control group (namely, less easily oxidized) (safflower < rapeseed < control).

**Table 1**

| group | Ingredients of feed |
|---|---|
| control | Normal diet (20% (w/w) vitamin-free casein, 66.3% starch, 5% corn oil, 3.5% AIN-93-mineral mixture, 1% AIN-93-vitamin mixture, 0.2% choline chloride, 4% cellulose powder) |
| rapeseed | Normal diet+1.3% (w/w) rapeseed meal extract* *balanced with starch |
| safflower | Normal diet+1.0% (w/w) safflower meal extract* *balanced with starch |

### Example 3

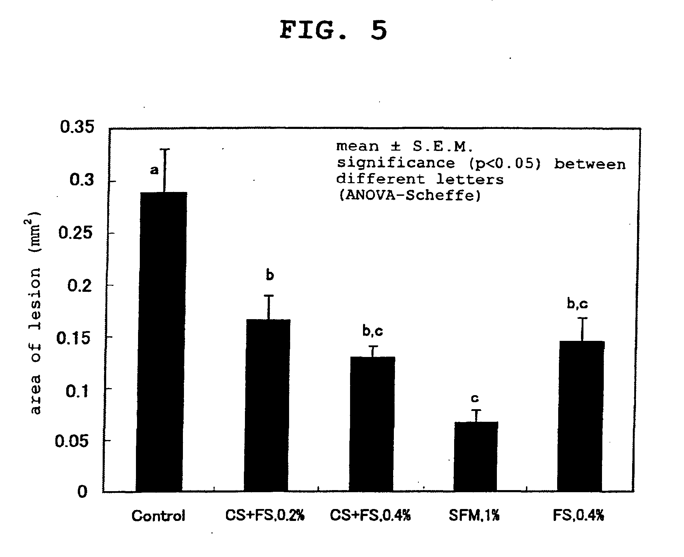

6-7-week-old male apoE knockout mice (purchased from The Jackson Laboratory) were divided into 5 group of control (Control)/serotonin derivative 0.2 wt% administration (p-coumaroylserotonin (CS), feruloylserotonin (FS), 0.1% each) (CS+FS, 0.2%)/serotonin derivative 0.4 wt% administration (p-coumaroylserotonin (CS), feruloylserotonin (FS), 0.2% each) (CS+FS, 0.4%)/feruloylserotonin (FS) 0.4 wt% administration (FS, 0.4%)/safflower meal extract (SFM) 1 wt% administration (SFM, 1%) with 7-10 mice per group, and each group was allowed free intake of the feed having ingredients shown in Table 2 for 15 weeks. The safflower meal extract (SFM) used in this Example was prepared according to the method shown in Example 2. After the completion of the administration period, the mice were killed, the aortic root was sliced and the lipid deposition part (atherosclerosis lesion) was stained with Oil Red O. Three slices were prepared for one individual and the samples most clearly showing the aortic valve were subjected to image analysis (using WinROOF (MITANI CORPORATION)) and the area of the lesion was measured based on the method of Rajendra et al (J. Lipid Res., 36: pp 2320-2328, 1995). The obtained area of the lesion was subjected to an analysis of variance between respective groups, and when a significant difference was observed, the average values were compared between groups by the Scheffe test. While a serotonin derivative (Zhang et al, Chem. Pharm. Bull., 44: pp 874-876, 1996, Kawashima et al, J. Interferon Cytokine Res., 18: pp 423-428, 1998), which is a main phenolic substance in safflower meal known to have antioxidant activity and anti-inflammatory activity in vitro, partially suppressed lesion formation in apoE knockout mice, a safflower meal extract (SFM, containing 10-30 wt% of serotonin derivative) was found to suppress stronger than that (Fig. 5).

**Table 2**

| | g (in 1 kg of diet) | | | | |
|---|---|---|---|---|---|
| composition | Control | CS+FS, 0.2% | CS+FS, 0.4% | SFM, 1% | FS, 0.4% |
| vitamin-free casein | 200.0 | 200.0 | 200.0 | 200.0 | 200.0 |
| corn starch | 632.5 | 630.5 | 628.5 | 622.5 | 628.5 |
| corn oil | 70.0 | 70.0 | 70.0 | 70.0 | 70.0 |
| mineral mixture (AIN-93G) | 35.0 | 35.0 | 35.0 | 35.0 | 35.0 |
| vitamin mixture (AIN-93G) | 10.0 | 10.0 | 10.0 | 10.0 | 10.0 |
| choline tartrate | 2.5 | 2.5 | 2.5 | 2.5 | 2.5 |
| cellulose powder | 50.0 | 50.0 | 50.0 | 50.0 | 50.0 |
| p-coumaroyl-serotonin | 0.0 | 1.0 | 2.0 | 0.0 | 0.0 |
| Feruloyl-serotonin | 0.0 | 1.0 | 2.0 | 0.0 | 4.0 |
| safflower meal | 0.0 | 0.0 | 0.0 | 10.0 | 0.0 |
| total | 1000.0 | 1000.0 | 1000.0 | 1000.0 | 1000.0 |

### Industrial Applicability

Since the composition of the present invention, which is obtained by organic solvent extraction of defatted plant seed is useful for preventing atherosclerosis, and is a naturally occurring material, it shows high safety and almost no side effects. A food and a pharmaceutical composition containing the composition are effective for preventing atherosclerosis.

This application is based on a patent application No. 110932/2002 filed in Japan, the contents of which are all hereby incorporated by reference.

## Claims

1. A composition for preventing atherosclerosis, which can be obtained by organic solvent extraction of a defatted plant seed.

2. The composition of claim 1, wherein the plant seed is a seed of safflower or rapeseed.

3. The composition of claim 1 or 2, wherein the organic solvent extraction comprises extracting a defatted plant seed with lower alcohol.

4. The composition of claim 3, wherein the organic solvent extraction comprises extraction with acetate ester after the extraction with lower alcohol.

5. The composition of claim 3 or 4, wherein the organic solvent extraction comprises, after the extraction with lower alcohol, evaporating lower alcohol, adding water and washing an aqueous phase with a nonpolar solvent.

6. The composition of any of claims 3 to 5, wherein the lower alcohol is ethanol or methanol.

7. The composition of any of claims 4 to 6, wherein the acetate ester is ethyl acetate, methyl acetate or propyl acetate.

8. The composition of any of claims 5 to 7, wherein the nonpolar solvent is n-hexane.

9. A food comprising the composition of any of claims 1 to 8.

10. A pharmaceutical composition comprising the composition of any of claims 1 to 8.
